Europäisches Patentamt

European Patent Office

Office européen des brevets

(11) Publication number: **0 450 461 B1**

# EUROPEAN PATENT SPECIFICATION

(45) Date of publication of patent specification: **06.09.95**  (51) Int. Cl.[6]: **C07K 7/23**, A61K 38/09

(21) Application number: **91104730.6**

(22) Date of filing: **26.03.91**

The file contains technical information submitted after the application was filed and not included in this specification

(54) **LHRH Analogs.**

(30) Priority: **06.04.90 US 505517**

(43) Date of publication of application:
**09.10.91 Bulletin 91/41**

(45) Publication of the grant of the patent:
**06.09.95 Bulletin 95/36**

(84) Designated Contracting States:
**AT BE CH DE DK ES FR GB GR IT LI LU NL SE**

(56) References cited:
**WO-A- 0/09799**

**Bajusz et al., Proc Natl. Acad. Sc. 86, 6313-6317 (1989)**

**PROCEEDINGS OF THE NATIONAL ACADEMY OF SCIENCES OF USA. vol. 86, no. 16, 1989, WASHINGTON US pages 6318-6322;S.BAJUSZ E.A.:Highly potent analogues of luteinizing hormone-releasing hormone containing D-phenylalanine nitrogen mustard in position 6' the whole document**

(73) Proprietor: **The Administrators of The Tulane Educational Fund**
**1430 Tulane Avenue**
**New Orleans**
**Louisiana 70112 (US)**

(72) Inventor: **Schally, Andrew Victor**
**5025 Kawanee Avenue**
**Metairie**
**LA 70006 (US)**
Inventor: **Janaky, Tamas**
**Hullum u. 7sz, II/10**
**H-6721 Szeged (HU)**
Inventor: **Juhasz, Atilla**
**Petnehazy ut 23sz, 10/24**
**H-1139 Budapest (HU)**
Inventor: **Bajusz, Sandor**
**Derek ut 16A**
**H-1016 Budapest (HU)**

(74) Representative: **Dr. Fuchs, Dr. Luderschmidt Dr. Mehler, Dipl.-Ing. Weiss Patentanwälte**
**Postfach 46 60**
**D-65036 Wiesbaden (DE)**

journal of medicinal chemistry. VOL. 31, 1988, washington us PAGES 341-345;k.krowicki E.A.: 'Novel DNA groove binding alkylators: design,synthesis and biological evaluation' the whole document

JOURNAL OF MEDICINAL CHEMISTRY.vol. 23, 1980, WASHINGTON US pages 1237 - 1242;TAI-Shun LIN E.A.: '2-Methylanthraquinone derivatives as potential bioreductive alkylating agents' the whole document

WIDDER AND GREEN 'METHODS IN ENZYMOLOGY,VOL.112,DRUGS AND ENZYME TARGETING,PART A' 1985, ACADEMIC PRESS J.M:varga, 'HORMONE-DRUG CONJUGATES' page 259-269.

**Description**

The present invention relates to novel peptides which contain cytotoxic moieties, have influence on the release of gonadotropins from the pituitary in mammals and possess antineoplastic effect. More specifically, the present invention relates to analogs of luteinizing hormone-releasing hormone (LHRH) with the structure of

pGlu-His-Trp-Ser-Tyr-Gly-Leu-Arg-Pro-Gly-NH$_2$

salts thereof and to pharmaceutical compositions and methods of using these analogs.

DISCUSSION OF THE PRIOR ART

Hypothalamic luteinizing hormone-releasing hormone (LHRH) controls the pituitary release of gonadotropins (LH and FSH) that stimulate the synthesis of sex steroids in the gonads.

A new approach in the treatment of hormone-sensitive tumors has been developed directed to the use of agonists and antagonists of LHRH (A.V. Schally and A.M. Comaru-Schally, Sem. Endocrinol., 5 389-398, 1987). Some LHRH agonists, when substituted in position 6, 10, or both are much more active than LHRH and also possess prolonged activity. The following superagonists are used in the clinical practice:

[D-Leu$^6$,NH-Et$^{10}$]LHRH (Leuprolide; J.A. Vilchez-Martinez et al., Biochem. Biophys. Res. Commun., 59 1226-1232, 1974)

[D-Trp$^6$]LHRH (Decapeptyl, D. H. Coy et al., J.Med.Chem., 19 423-425, 1976).

[D-Ser(tBu)$^6$,NH-Et$^{10}$]LHRH (Buserelin, W. Koenig et al., In: R. Walter and J. Meienhofer (eds.),

Peptides: Chemistry, Structure and Biology. Proceedings of the Fourth American Peptide Symposium. Ann Arbor Science, Ann Arbor, MI, 1975, pp. 883-888.

[D-Ser(tBu)$^6$,NH-NH-CO-NH$_2$$^{10}$]LHRH (Zoladex, A.S. Dutta et al., J. Med. Chem., 21 1018-1024, 1978).

[D-Nal(2)$^6$]LHRH (Nafarelin, J.J. Nestor et al., J. Med. Chem., 25 795-801, 1982).

Changes in position 1, 2, 3, 6 and optionally in positions 5 and 10 of the LHRH molecule led to the creation of powerful antagonists (M.J. Karten and J.E. Rivier, Endocrine Review, 7 44-66, 1986; S. Bajusz et al., Int. J. Pept. Prot. Res., 32 425-435, 1988) which inhibit the LH and FSH release from the pituitary and have potential as therapeutic agents in the treatment of hormone dependent cancers (prostate, breast and pancreatic) (A.V. Schally, in General Gynecology, Vol 6., Parthenon Press, Carnforth, England, 1989, pp. 1-20).

Ideal anticancer drugs would theoretically be those that eradicate cancer cells without harming normal cells. Hormones carrying antineoplastic agents would solve the problem by achieving more efficiently targeted chemotherapy of receptor-containing tumors. An ideal mechanism of action of hormone-drug conjugates would be their binding to a cell membrane receptor, followed by internalization of the hybrid molecules and release of the drugs or their biologically active derivatives from the carrier hormone in the endosomes or secondary lysosomes. The released substances then pass across the membrane of the vesicles into the cytosol and reach their final target sites. For the conjugates to be effective by this mechanism, the bond between the drug and hormone must be stable before internalization of conjugates into the target tumor cells but should be effectively cleaved after this internalization.

Many human tumors are hormone dependent or hormone-responsive and contain hormone receptors. Certain of these tumors are dependent on or responsive to sex hormones or growth factors or have components which are so dependent or responsive. The remaining tumors or tumor components are not so dependent. Mammary carcinomas contain estrogen, progesterone, glucocorticoid, LHRH, EGF, IGF-I. and somatostatin receptors. Peptide hormone receptors have also been detected in acute leukaemia, prostate-, breast-, pancreatic, ovarian-, endometrial cancer, colon cancer and brain tumors (M.N. Pollak, et al., Cancer Lett. 38 223-230, 1987; F. Pekonen, et al., Cancer Res., 48 1343-1347, 1988; M. Fekete, et al., J. Clin.Lab. Anal. 3 137-147, 1989; G. Emons, et al., Eur. J. Cancer Oncol., 25 215-221, 1989). It has been found (M. Fekete, et al., Endocrinology, 124 946-955, 1989; M. Fekete, et al.Pancreas 4 521-528, 1989) that both agonistic and antagonistic analogs of LHRH bind to human breast cancer cell membranes, and also to the cell membranes of pancreatic cancer, although the latter tumor thought to be hormone-independent. It has been demonstrated that biologically active peptides such as melanotropin (MSH), epidermal growth factor, insulin and agonistic and antagonistic analogs of LHRH (L. Jennes, et. al., Peptides 5 215-220, 1984) are internalized by their target cells by endocytosis.

Alkylating agents used in the treatment of cancer have a basically nonselective mechanism of action. They act by exerting the cytotoxic effect via transfer of their alkyl groups to various cell constituents.

Alkylation of DNA within the nucleus probably represents the major interaction that leads to cell death. However, under physiologic conditions, one can alkylate all cellular nucleophiles such as ionized carboxylic and phosphoric acid groups, hydroxyl groups, thiols and uncharged nitrogen moieties. Nitrogen mustards (chlorambucil, cyclophosphamide and melphalan) are among the oldest anticancer drugs in clinical use. They spontaneously form cyclic aziridinium (ethylenimonium) cation derivatives by intramolecular cyclization, which may directly or through formation of a carbonium ion, transfer an alkyl group to a cellular nucleophile. Aziridine moiety containing drugs like thio-TEPA act by the same mechanism.

Cyclopropane is another alkylating agent. The highly strained ring is prone to cleavage by nucleophiles. It can be cleaved to singlet biradical transition and zwitterion transition state in epimerization reactions and thus might act as an alkylating species for interaction with nucleophilic bases of DNA. Incorporation of cyclopropyl group into distamycin (natural antiviral antitumor agent) resulted in four fold increase in cytostatic activity (K. Krowicki, et al., J. Med. Chem. 31 341-345, 1988).

Almost all clinically used alkylating agents are bifunctional and have ability to cross-link two separate molecules, or alkylate one molecule at two separate nucleophilic sites. The cross-links with DNA may be within a single strand, between two complementary strands or between DNA and other molecules, such as proteins. It is thought that the cytotoxicity of alkylating agents is correlated with their cross-linking efficiency (J.J. Roberts et al., Adv. Radiat. Biol. 7 211-435, 1978).

Cisplatin (cis-diaminedichloroplatinum) has been used in the cancer therapy for a long time. LHRH analogs with cisplatin related structure in the side-chain have high affinities for membrane receptors of rat pituitary and human breast cancer cells (S. Bajusz et al. Proc. Natl. Acad. Sci. USA 86 6313-6317, 1989). Incorporation of cytotoxic copper(II) and nickel(II) complexes into suitably modified LHRH analogs resulted in compounds with high hormonal activity and affinity for LHRH receptors on human breast cancer cell membrane. Several of these metallopeptides have cytotoxic activity against human breast and prostate cell lines in vitro. For example pGlu-His-Trp-Ser-Tyr-D-Lys[Ahx-$A_2$bu(SAL)$_2$(Cu)]-Leu-Arg-Pro-Gly-NH$_2$ inhibits the [$^3$H]thymidine incorporation into DNA of the human mammary cell line MDA-MB-231 by 87% at 10$\mu$g dose.

Many drugs used in cancer chemotherapy contain the quinone group in their structure. Anthracycline antitumor antibiotics such as adriamycin, daunorubicin, mitomycin C and mitoxantrone bind to DNA through intercalation between specific bases and block the synthesis of new RNA or DNA (or both), cause DNA strand scission, and interfere with cell replication. Bioreductive reactions of the quinone group can lead to formation of free radicals (superoxide and hydroxyl radicals) that can induce DNA strand breaks (Bachur et al. Cancer Res. 38 1745-1750, 1978). An alternative pathway is the reduction of quinone to hydroquinone followed by conversion into the alkylating intermediate, the quinonemethide (Moore et al., Drug Exp. Clin. Res. 12 475-494, 1986). Daunorubicin was coupled to peptide carrier melanotropin (MSH) and the conjugate proved to be more toxic to murine melanoma cells than free drug (J.M. Varga, Meth. Enzymol. 112 259-269, 1985). 2-Methylanthraquinone derivatives have cytotoxic activity on hypoxic neoplastic cells (T.S. Lin, et al. J. Med. Chem. 23 1237-1242, 1980).

Several antimetabolites are of potential chemotherapeutic interest because of their importance in cellular folate metabolism (I.D. Goldman, et al., Eds. Folyl and Antifolyl Polyglutamates. Plenum press, New York, 1983.). Methotrexate {N-[p[[(2,4-diamino-6-pteridinyl)methyl]methylamino]benzoyl]glutamic acid is a folic acid antagonist that inhibits the function of dihydrofolate reductase and in this way interrupts the synthesis of thymidilate, purine nucleotides, and the amino acids serine and methionine, thereby interfering with the formation of DNA, RNA, and proteins.

Initially the incorporation of the alkylating drug chlorambucil {4-[4-(bis[2-chloroethyl]amino)phenyl}-butyric acid into LHRH agonist and antagonists led to compounds with low activity or no activity [K. Channabasavaiah and J. M. Stewart, Biochem. Biophys. Res. Commun., 86, 1266-1273 (1979), C. Y. Bowers et al., Biochem. Biophys. Res. Commun., 61, 698-703 (1974), K. Channabasavaiah et al., In: E. Gross and J. Meienhofer (eds.), Peptides, Proceedings of the Sixth American Peptide Symposium, Pierce Chem. Co. Rockford, IL, 1979, pp 803-807].

D-melphalan (a nitrogen mustard type alkylating agent, 4-[bis{2-chloroethyl}amino]-D-phenylalanine) containing LHRH analogs have high agonistic and antagonistic activity and bind to the rat pituitary, human breast and prostate cancer cell membranes with high affinity (S. Bajusz et al., Proc. Natl. Acad. Sci. USA 86 6318-6322, 1989). The binding is reversible and no alkylation of the LHRH receptors occurred. Significant cytotoxic activity (inhibition of [$^3$H]thymidine incorporation) in cultures of human breast cancer cell line T-47D and rat mammary tumor cell line MT-4 and MT-5 could be demonstrated.

## SUMMARY OF THE INVENTION

Sex hormone and growth factor dependent tumors or tumor components may be suppressed by lowering the levels of these factors in the patient's system. This does not however, deal with the problem of the remaining non-dependent tumors or tumor components. As shown by Fekete and others (supra), LHRH receptors are either present or appear in tumors and tumor components not dependent on sex hormone or growth factors.

Thus, LHRH analogs containing a cytotoxic moiety might serve as carriers for the chemotherapeutic agents. Such peptides can bind to LHRH receptors and not destroy the receptor site, this might provide some target selectivity for the thus modified cytotoxic LHRH analog and make it "cell specific". After internalization, the cytotoxic component of these hybrid compounds could interfere with cellular events and thus cause cancer cell death.

There are several compounds among the clinically used anticancer drugs which have the potential of being coupled to a carrier peptide molecule. The coupling can be carried out through modification of the functional group of the cytotoxic moiety and the free amino- or carboxyl-group of a peptide.

The present invention deals with the provision of such LHRH analogues which possess high agonistic or antagonistic activity and contain cytotoxic side chains, such as moieties with quinone structure (substituted anthraquinones suitably by lower alkyl from which these moieties are derived), and antimetabolites like methotrexoyl. The majority of compounds significantly inhibit the growth of different human breast cancer cell lines in cell cultures.

The compounds of this invention are represented by Formula I

$$X-R^1-R^2-R^3-Ser-R^5-R^6(Q)-Leu-Arg-Pro-R^{10}-NH_2 \qquad I$$

wherein
$R^1$ is pGlu or D-Nal(2),
$R^2$ is His or, provided that $R^1$ is D-Nal(2), $R^2$ is D-Phe(4 Cl),
$R^3$ is Trp or, provided that $R^4$ is D-Nal(2), $R^3$ is D-Trp or D-Pal(3),
$R^5$ is Tyr or, provided that $R^1$ is D-Nal(2), $R^5$ is Arg.
$R^6$ is D-Lys
$R^{10}$ is Gly or, provided that $R^1$ is D-Nal(2), $R^{10}$ is D-Ala,
X is hydrogen or, provided that $R^1$ is D-Nal(2), X is acetyl,
Q is a cytotoxic moiety having the formula
$-Q^4$    II or $-A Q^2$    III or, provided that $R^1$ is D-Nal(2), Q is $-B(AQ^2)_2$    IV
wherein
A is glutaryl
B is 2,3-diaminopropionyl
the -CO moiety of A- and of B- being bonded to an amino group on $R^6$, and in the group $B(AQ^2)_2$, the -CO moiety of A- being bonded to an amino group on B,
$Q^2$ is anthraquinonylmethoxy,
$Q^4$ is methotrexoyl and
the pharmaceutically acceptable acid and base addition salts thereof.

The compounds of Formula I can be prepared by a combination of the solid phase technique and the classical (solution) synthesis.

Compounds of Formula I are preferably prepared from intermediate peptides of Formula VI:

$$X^1-R^1-R^2(X^2)-R^3-Ser(X^4)-R^5(X^5)-R^6(X^5)-Leu-Arg(X^5)-Pro-R^{1\circ}-NH-X^{10} \qquad VI$$

wherein
$R^1, R^2, R^3, R^5, R^6$ and $R^{10}$ are as defined above,
$X^1$ is an acetyl group or, provided that $R^1$ is pGlu, $X^1$ is hydrogen,
$X^2$ is nil or a protecting group for His imidazole nitrogen,
$X^4$ is hydrogen or a protecting group for the Ser hydroxyl group,
$X^5$ is hydrogen or a protecting group for the Tyr phenolic hydroxyl group, or a protecting group for the guanidino group of Arg,
$X^5$ is hydrogen or a protecting group for the Lys,
$X^5$ is hydrogen or a protecting group for the Arg guanidino group,
$X^{10}$ is hydrogen or benzhydryl group incorporated into a resin.

Peptides of Formula VI are preferably synthesized by solid phase method.

Intermediate peptides of Formula VII obtained from peptides of Formula VI, wherein $X^2$, $X^4$, $X^5$, $X^5$, $X^5$, and $X^{10}$ are hydrogen, by acylation with A:

$$X^1\text{-}R^1\text{-}R^2\text{-}R^3\text{-Ser-}R^5\text{-}R^6(A)\text{-Leu-Arg-Pro-}R^{10}\text{-}NH_2 \qquad VII$$

wherein $X^1$, $R^1$, $R^2$, $R^3$, $R^5$, $R^6$, $R^{10}$ and A are as defined above.

The acylation of peptides of Formula VI wherein $X^2$, $X^4$, $X^5$, $X^5$, $X^5$, and $X^{10}$ are hydrogen with suitably protected B gives after deprotection, intermediate peptides of Formula VIII:

$$X^1\text{-}R^1\text{-}R^2\text{-}R^3\text{-Ser-}R^5\text{-}R^6(B)\text{-Leu-Arg-Pro-}R^{10}\text{-}NH_2 \qquad VIII$$

wherein $X^1$, $R^1$, $R^2$, $R^3$, $R^5$, $R^6$, $R^{10}$ and $\underline{B}$ are as defined above.

According to another suitable method, intermediate peptides of Formula VIII are obtained by deprotection of intermediate peptides of Formula VIIIA:

$$X^1\text{-}R^1\text{-}R^2(X^2)\text{-}R^3\text{-Ser}(X^4)\text{-}R^5(X^5)\text{-}R^6[A(X^{6'})_2]\text{-Leu-Arg}(X^5)\text{-Pro-}R^{10}{}^-\text{NH-}X^{10} \qquad VIIIA$$

wherein $X^{6'}$ is hydrogen or a protecting group for the diaminoacid side chain, $R^1$, $R^2$, $R^3$, $R^5$, $R^6$, $R^{10}$, A, $X^1$, $X^2$, $X^4$, $X^5$, $X^5$, $X^5$, and $X^{10}$ are as defined above, which in turn are prepared by the solid phase method as intermediate peptides of Formula VI with the exception that suitably protected $R^6[B(X^5)_2]$ is incorporated in place of protected $R^6(X^5)$ in position 6.

To produce compounds of Formula I wherein Q is $B(AQ^2)_2$, peptides of Formula VIII were coupled with preformed $(AQ^2)$ wherein A and $Q^2$ are as defined above. Alternatively, compounds of Formula I wherein Q is $B(AQ^2)_2$, can be prepared by reacting peptides of Formula VIII first with an acylating agent with an A moiety and then with 2-hydroxymethylanthraquinone.

The synthesis of compounds of Formula I wherein Q is $A(Q^2)$ was carried out by elongation of the D-Lys side chain of peptides of Formula VI with glutaric acid and then coupling with 2-hydroxymethyl anthraquinone. Alternatively, compounds of Formula I wherein Q is $A(Q^2)$, can be prepared by reacting peptides of Formula VI with preformed $A(Q^2)$, where A and Q are as defined above.

The process of preparing compounds of Formula I wherein Q is $Q^4$ comprises reacting a peptide of Formula VI with methotrexate. Suitably, the reaction is carried out when $X^1$ is hydrogen and all other X moieties are hydrogens.

A pharmaceutical composition is provided by admixing the compound of Formula I with pharmaceutically acceptable carrier including microcapsules (microspheres) or microgranules (microparticles) formulated from poly(DL-lactide-co-glycolide) for sustained delivery.

## DESCRIPTION OF THE PREFERRED EMBODIMENTS

For convenience in describing this invention, the conventional abbreviations for the amino acids, peptides and their derivatives are used as generally accepted in the peptide art and as recommended by the IUPAC-IUB Commission on Biochemical Nomenclature [European. J. Biochem., 138, 9-37 (1984)].

The abbreviations for the individual amino acid residues are based on the trivial name of the amino acid, e.g. pGlu is pyroglutamic acid, His is histidine, Trp is tryptophan, Ser is serine, Tyr is tyrosine, Lys is lysine, Orn is ornithine, Leu is leucine, Arg is arginine, Pro is proline, Gly is glycine, Ala is alanine and Phe is phenylalanine. Where the amino acid residue has isomeric forms, it is the L-form of the amino acid that is represented unless otherwise indicated.

Abbreviations of the uncommon amino acids employed in the present invention are as follows: $A_2pr$ is 2,3-diaminopropionic acid, D-Nal(2) is 3-(2-naphthyl)-D-alanine, D-Pal(3) is 3-(3-pyridyl)-D-alanine, D-Phe-(4Cl) is 4-chloro-D-phenylalanine.

Peptide sequences are written according to the convention whereby the N-terminal amino acid is on the left and the C-terminal amino acid is on the right.

Other abbreviations used are:

AcOH acetic acid
$Ac_2O$ acetic anhydride
Azy aziridin-2-carbonyl
Boc tert.butoxycarbonyl
Bzl benzyl

6

DCB 2,6-dichlorobenzyl
DCC N,N'-dicyclohexylcarbodiimide
DCM dichloromethane
DIC N,N'-diisopropylcarbodiimide
DMF dimethylformamide
EPP epoxy-propyl
HMAQG anthraquinone-2-methylglutarate
HOBt 1-hydroxybenzotriazole
HOPCP pentachlorophenol
HPLC high-performance liquid-chromatography
MeCN acetonitrile
MeOH methyl alcohol
MTX methotrexate (amethopterin)
TEA triethylamine
TFA trifluoroacetic acid
THF tetrahydrofuran
Tos 4-toluenesulfonyl
Z(2-Cl) 2-chloro-benzyloxycarbonyl
Z benzyloxycarbonyl

Especially preferred are LHRH analogues of Formula I

$$X-R^1-R^2-R^3-Ser-R^5-R^6(Q)-Leu-Arg-Pro-R^{10}-NH_2 \qquad I$$

wherein,
$R^1$ is D-Nal(2),
$R^2$ is D-Phe(4Cl),
$R^3$ is D-Trp or D-Pal(3),
$R^5$ is Arg,
$R^6$ is D-Lys,
$R^{10}$ is D-Ala,
X is acetyl, as well as peptide series, where
$R^1$ is pGlu,
$R^2$ is His,
$R^3$ is Trp,
$R^5$ is Tyr,
$R^6$ is D-Lys
$R^{10}$ is Gly, and
X is hydrogen,
Q is a cytotoxic moiety having the formula:
$Q^4$ or $B(AQ^2)_2$
A is glutaric acid residue
B is $A_2pr$,
$Q^2$ is anthraquinone-2-methoxy
$Q^4$ is methotrexoyl.
The most particularly preferred embodiments are:
1. pGlu-His-Trp-Ser-Tyr-D-Lys(HMAQG)-Leu-Arg-Pro-Gly-NH$_2$
2. pGlu-His-Trp-Ser-Tyr-D-Lys(MTX)-Leu-Arg-Pro-Gly-NH$_2$
3. Ac-Nal(2)-D-Phe(4Cl)-D-Trp-Ser-Arg-D-Lys[A$_2$pr(HMAQG)$_2$]-Leu-Arg-Pro-D-Ala-NH$_2$
4. Ac-Nal(2)-D-Phe(4Cl)-D-Pal(3)-Ser-Arg-D-Lys[A$_2$pr(HMAQG)$_2$]-Leu-Arg-Pro-D-Ala-NH$_2$

The peptides of the invention may be used for the preparation of a composition to be administered in the form of pharmaceutically acceptable, nontoxic salts, such as add addition salts. Illustrative of such acid addition salts are hydrochloride, hydrobromide, sulphate, phosphate, fumarate, gluconate, tannate, maleate, acetate, citrate, benzonate, succinate, alginate, pamoate, malate, ascorbate, tartrate, and the like.

Microcapsules or microparticles of these peptides formulated from poly(DL-lactide-co-glycolide) may be the preferred sustained delivery systems. The peptides may also be used for the preparation of a composition destined for intravenous administration in isotonic saline, phosphate butter solutions or the like.

The pharmaceutical compositions will usually contain the peptide in conjunction with a conventional, pharmaceutically-acceptable carrier. Usually, the dosage will be from about 1 to about 100 micrograms of the peptide per kilogram of the body weight of the host when given intravenously.

These peptides can be used for the preparation of a composition to be administered to mammals intravenously, subcutaneously, intramuscularly, intranasally or intravaginally to achieve antitumor effect. Effective dosages will vary with the form of administration and the particular species of mammal being treated. An example of one typical dosage form is a physiological saline solution containing the peptide which solution is destined to provide a dose in the range of about 0.1 to 2.5 mg/kg of body weight.

Although the invention has been described with regard to its preferred embodiments, it should be understood that changes and modifications obvious to one having the ordinary skill in this art may be made without departing from the scope of the invention, which is set forth in the claims which are appended thereto. Substitutions known in the art which do not significantly detract from its effectiveness may be employed in the invention.

## Assay procedures

The compounds of this invention exhibit powerful effect on gonadotropin release by the pituitary, bind to tumor cell membranes and inhibit [³H]thymidine incorporation into DNA in cell cultures.

### (a) LH-releasing and LH-RH-inhibiting activities

Ability of compounds to influence LH release in vitro is assayed by using a superfused rat pituitary cell system [S. Vigh and A. V. Schally, Peptides, 5 Suppl. 1, 241-247 (1984); V. Csernus and A.V. Schally, in Neuroendocrine Research Methods, Ed. B. Greenstein, Harwood Academic Publishers, London, (1990)].

LH-releasing effect of compounds is determined as follows: each peptide is perfused through the cells for 3 min (1 ml perfusate) at 20-100 pM. LH content of 1 ml fractions collected is determined by radioimmunoassay (RIA). Potency of peptides is compared to that of 3 nM LHRH perfused similarly.

LHRH inhibiting effect of peptides is assayed as follows: each peptide is perfused through the cells for 9 min (3 ml perfusate) at 1 nM. Immediately after that, a mixture containing the same concentration of peptide and 3 nM LHRH is administered for 3 min. This was followed by four consecutive infusions of 3 nM LHRH for 3 min (1 ml perfusate) at 30 min intervals (30, 60, 90, 120 min). LH content of the 1 ml fractions collected is determined by RIA.

### (b) In vivo antiovulatory activity

This activity of the peptides is determined in 4-day-cycling rats as described [A. Corbin and C. W. Beattie, Endocr. Res. Commun., 2, 1-23 (1975)].

### (c) Receptor binding.

Affinity for peptides to human breast cancer cell membranes is determined by using labelled LHRH and [D-Trp⁶]LHRH. The assay is carried out similarly to that described by T. Kadar et al., Proc. Natl. Acad. Sci. USA, 85, 890-894 (1988) and M. Fekete et al., Endocrinology, 124, 946-955 (1989).

### (d) Cytotoxicity test.

Ability of peptides of Formula I to inhibit incorporation of [³H]thymidine into DNA of monolayer cultures the human mammary tumor cell line MCF-7 is assayed as described [V. K. Sondak et al., Cancer Research, 44, 1725-1728 (1984); F. Holzel et al., J. Cancer Res. Clin. Oncol. 109, 217-226 (1985); M. Albert et al., J. Cancer Res. Clin. Oncol. 109, 210-216 (1985)].

## Synthesis of peptides

The peptides of the present invention may be prepared by any techniques that are known to those skilled in the peptide art. A summary of the techniques so available may be found in M. Bodanszky, Principles of Peptide Synthesis, Springer-Verlag, Heildelberg, 1984. Classical solution synthesis is described in detail in the treatise "Methoden der Organische Chemie" (Houben-Weyl), Vol. 15, Synthese von Peptiden, Parts I and II, Georg Thieme Verlag, Stuttgart, 1974. The techniques of exclusively solid-phase synthesis are set forth in the textbook of J. M. Stewart and J. D. Young, Solid Phase Peptide Synthesis, Pierce Chem Co., Rockford, IL, 1984 (2nd ed.) and in the review of G. Barany, et al., Int. J. Peptide Protein Res. 30, 705-739, 1987.

The basic peptides of this invention were synthesized by solid-phase method, and only the cytotoxic side chains were incorporated by "classical" procedure. In the solid phase synthesis, suitable protected amino acids (sometimes protected peptides) are added stepwise in C-->N direction once the C-terminal amino acid has been appropriately attached (anchored) to an inert solid support (resin). After completion of a coupling step, the N-terminal protecting group is removed from this newly added amino acid residue and the next amino acid (suitably protected) is then added, and so forth. After all the desired amino acids have been linked in the proper sequence, the peptide is cleaved from the support and freed from the remaining protecting group(s) under conditions that are minimally destructive towards residues in the sequence. This must be followed by a prudent purification and scrupulous characterization of the synthetic product, so as to ensure that the desired structure is indeed the one obtained.

**Preferred Embodiment of Synthesis**

A particularly preferred method of preparing compounds of the present invention is the solid phase synthesis; the incorporations of cytotoxic side chains are performed in solution. The peptides of Formula I wherein $R^6$ is D-Lys are preferably prepared from intermediate peptides of Formula VI:

$X^1$-$R^1$-$R^2$($X^2$)-$R^3$-Ser($X^4$)-$R^5$($X^5$)-$R^6$($X^5$)-Leu- Arg($X^5$)-Pro-$R^{10}$-NH-$X^{10}$      VI

wherein
$R^1$, $R^2$, $R^3$, $R^5$, $R^6$, $R^{10}$ and $X^1$ are as defined hereinabove,
$X^2$ is p-toluenesulfonyl or 2,4-dinitrophenyl protecting group if $R^2$ is His and nil if $R^2$ is D-Phe(4Cl),
$X^4$ is a protecting group for the hydroxyl group of serine, such as benzyl (Bzl) or 2,6-dichlorobenzyl (DCB). The preferred protecting group is Bzl.
$X^5$ is benzyl, 2-Br-benzyloxycarbonyl or DCB (preferred) for protecting the phenolic hydroxyl where $R^5$ is Tyr, or is Tos (preferred), nitro or methyl-(t-butylbenzene)-sulfonyl to protect the guanidino group if $R^5$ is Arg,
$X^5$ is a protecting group for side chain amino group of Lys, such as Z, Z(2-Cl) (preferred),
$X^5$ is suitable group to protect the Arg: nitro, methyl-(t- butylbenzene)-sulfonyl or Tos (preferred),
$X^{10}$ is an amide protecting benzhydryl or methylbenzhydryl group incorporated into resin support; for synthesis of peptide amides, the commercially available benzhydrylamino- polystyrene-2% divinylbenzene copolymer is preferred.

The solid phase synthesis of the peptides of Formula VI is commenced by the attachment of Boc-protected Gly or D-Ala to a benzhydrylamine resin in $CH_2Cl_2$. The coupling is carried out using DIC or DIC/HOBt at ambient temperature. After the removal of the Boc group, the coupling of successive protected amino acids (each is applied in a 3 molar excess) is carried out in $CH_2Cl_2$ or in mixtures of DMF/$CH_2Cl_2$ depending on the solubility of Boc-amino acids. The success of the coupling reaction at each stage of the synthesis is preferably monitored by the ninhydrin test as described by Kaiser et al. [Anal. Biochem. 34, 595 (1970)]. In cases where incomplete coupling occurs, the coupling procedure is repeated before removal of the alpha-amino protecting group prior to the reaction with the next amino acid.

After the desired amino acid sequence of intermediate peptides of Formula VI has been completed, if desired, the N-terminal acetylation is carried out using $Ac_2O$/imidazole, and the peptide-resin is then treated with liquid HF in the presence of anisole to yield the peptides of Formula VI wherein $X^2$, $X^4$, $X^5$, $X^5$, $X^5$, and $X^{10}$ are hydrogens.

Peptides of Formula VII were obtained either by acylation of peptides of Formula VI with glutaric anhydride, followed by deprotection:

$X^1$-$R^1$-$R^2$-$R^3$-Ser-$R^5$-$R^6$(A)-Leu-Arg-Pro-$R^{10}$-NH$_2$      VII

wherein $X^1$, $R^1$, $R^2$, $R^3$, $R^5$, $R^6$, and $R^{10}$ are as defined above, and A is glutaryl.

Acylation of peptides of structure of Formula VI with Boc-protected 2,3-diamino propionic acid, after deprotection gives the peptides of Formula VIII:

$X^1$-$R^1$-$R^2$-$R^3$-Ser-$R^5$-$R^6$(B)-Leu-Arg-Pro-$R^{10}$-NH$_2$      VIII

wherein $X^1$, $R^1$, $R^2$, $R^3$, $R^5$, $R^6$, and $R^{10}$ are as defined hereinabove, and B is 2,3-diamino propionyl.

In an alternate synthesis, peptides of Formula VIII are obtained by deprotection of intermediate peptides of Formula VIIIA which are prepared by the solid phase method exactly as peptides having the Formula VI, but a suitably protected $R^6(B)$ residue, preferably Boc-$R^6[B(Z)_2]$, incorporated in position 6 instead of Boc-$R^6 X^6$.

Compounds of Formula I wherein residue Q is $B(AQ^2)_2$ are preferably prepared from intermediate peptides of Formula VIII and 2-hemiglutaroyl-oxymethyl-anthraquinone coupling them together in a reaction with carbodiimide.

To produce compounds of Formula I wherein Q is $Q^4$, MTX is bound to intermediate peptides of Formula VII by carbodiimide reaction.

Peptides of Formula VI are converted into peptides of Formula I wherein Q is $Q^4$ by carbodiimide coupling method with 1.1 equivalent of MTX.

## PURIFICATION OF PEPTIDES

Crude synthetic products (>500 mg) were purified on a BECKMAN Prep-350 preparative HPLC system equipped with a DYNAMAX MACRO column (41.4 x 250 mm) packed with spherical C18 silica gel (pore size: 300 Å, particle size: 12 $\mu$m) (RAININ Inc., Co., Woburn, MA) (**Column A**). Purification of smaller amount of peptides (<250 mg) were performed on a BECKMAN HPLC system (Model 142) using a DYNAMAX MACRO (21.2 x 250 mm) column packed with the same medium, as above (**Column B**). To purify peptides weighing <50 mg, a reversed phase, 10 x 250 mm VYDAC Protein & Peptide $C_{18}$ column (pore size: 300 Å, particle size: 5 $\mu$m) (ALTECH, Deerfield, IL) (**Column C**) or a 10 x 250 mm W-POREX $C_{18}$ column (pore size: 300 Å, particle size: 5 $\mu$m) (Phenomenex, Rancho Palos Verdes, CA) (**Column D**) were used. Columns were eluted with solvent system i consisting of (A) 0.1% aqueous TFA and (B) 0.1% TFA in 70% aqueous acetonitrile or solvent system ii consisting of (A) 0.2% aqueous acetic acid and (B) 0.2% acetic acid in 70% aqueous acetonitrile usually in a gradient mode. Column eluant was monitored with UV detectors operating at 230 or 280 nm. Chromatography was effected at ambient temperature.

## ANALYTICAL HPLC

Analysis of crude and purified peptides was carried out with a Hewlett-Packard Model 1090 liquid chromatograph equipped with a diode array detector set a 220 and 280 nm and a reversed phase 4.6 X 250 mm W-POREX $C_{18}$ column (pore size: 300 Å, particle size: 5 $\mu$m) (**Column E**). A flow rate of 1.2 ml/min of solvent system i was maintained and the separations were performed at room temperature.

## AMINO ACID ANALYSIS

Peptide samples were hydrolyzed at 110°C for 20 hr in evacuated sealed tubes containing 4 M methane-sulfonic acid. Analyses were performed with a Beckman 6300 amino acid analyzer.

## PREPARATION I

pGlu-His-Trp-Ser-Tyr-D-Lys-Leu-Arg-Pro-Gly-NH$_2$      (I)

[D-Lys]$^6$LHRH [N.C. Nicholas et al., J. Med. Chem., 19 937-941 (1976)] was built step by step on a benzhydrylamine HCl resin containing about 1 meq NH$_2$/g (Advanced ChemTech, Louisville, KY) in a reaction vessel for manual solid-phase synthesis starting with Boc-Gly in accordance with the procedures set forth below.

The benzhydrylamine HCl resin (1 g, about 1 mmol), after neutralization with 10% TEA in CH$_2$Cl$_2$, was coupled sequentially with a 3 molar excess of protected amino acids in accordance with the Schedule as follows:

| STEP | REAGENTS AND OPERATIONS | MIXING TIMES (min) |
|---|---|---|
| 1 | Coupling: Boc-amino acid in DCM or DMF depending on the solubility of the particular protected amino acid, plus DIC | 60-90 |
| 2 | MeOH (or DMF then MeOH) wash | 2 |
| 3 | DCM wash | 2 |
| 4 | MeOH wash | 2 |
| 5 | DCM wash (three times) | 2 |
| 6 | Deprotection: 50% TFA in DCM (twice) | 5 and 25 |
| 7 | DCM wash | 2 |
| 8 | 2-Propanol wash | 1 |
| 9 | Neutralization: 10% TEA in DCM | 2 |
| 10 | MeOH wash | 1 |
| 11 | Neutralization: 10% TEA in DCM | 2 |
| 12 | MeOH wash | 1 |
| 13 | DCM wash (three times) | 2 |

Thus, the resin was treated with Boc-Gly, Boc-Pro, Boc-Arg(Tos), Boc-Leu, Boc-D-Lys[Z(2-Cl)], Boc-Tyr(Bzl), Boc-Ser(Bzl), Boc-Trp, Boc-His(Tos), and pGlu during successive coupling cycles to yield a peptide-resin with structure of pGlu-His(Tos)-Trp-Ser(Bzl)-Tyr(DCB)-D-Lys[Z(2-Cl)]-Leu-Arg(Tos)-Pro-Gly-NH-RESIN.

The peptide-resins thus obtained were treated with 2 ml anisole and 20 ml of HF at 0° for 45 min. After elimination of HF under vacuum, the peptide-resin remainder was washed with dry diethyl ether. The peptide was then extracted with 50% aqueous acetic acid, separated from the resin by filtration, and lyophilized.

Crude peptide (860 mg, was purified on Column A with solvent system $\underline{i}$ using a linear gradient of 10-40 % B in 60 min at flow rate of 30 ml/min. 230 nm.

Purified peptide proved to be substantially (>96%) pure in analytical HPLC by using solvent system $\underline{i}$ in a linear gradient mode (15-35%B in 20 min). Retention time is 11.3 min. Amino acid analysis gave the expected results.

## PREPARATION II

Ac-D-Nal(2)-D-Phe(4Cl)-D-Trp-Ser-Arg-D-Lys-Leu-Arg-Pro-D-Ala-NH$_2$        (II)

The preparation of II was carried out by solid-phase method in accordance with the procedures set forth in the Schedule of Preparation I. The synthesis was commenced by coupling Boc-D-Ala to 1 g benzhydrylamine resin containing about 1.0 meq NH$_2$. The decapeptide was built up in nine successive coupling steps using Boc-Pro, Boc-Leu, Boc-Arg(Tos), Boc-Lys[Z(2-Cl)], Boc-Tyr(DCB), Boc-Ser(Bzl), Boc-D-Trp, Boc-D-Phe(4Cl), Boc-D-Nal(2). N-Terminal acetylation was performed with a 50-fold excess of acetic anhydride in CH$_2$Cl$_2$ for 30 min. The peptide was cleaved from the resin with 15 ml of HF in the presence of 1.5 ml m-cresol at 0 °C for 30 min and at room temperature for 30 min. After elimination of HF, the mixture of resin and peptide was washed with diethyl ether, the peptide was extracted with DMF. The DMF solution was concentrated to a small volume under high vacuum, then triturated with diethylether. The crude product thus obtained was purified by preparative HPLC as described for Preparation I, using a linear gradient of 40-70%B in 60 min. The pure peptide (837 mg) has a retention time of 25.5 min using solvent system $\underline{i}$ in a linear gradient mode (30-60%B in 30 min).

## PREPARATIONS III

Ac-D-Nal(2)-D-Phe(4Cl)-D-Pal(3)-Ser-Arg-D-Lys-Leu-Arg-Pro-D-Ala-NH$_2$        (III)

The peptide IIIA was prepared by the solid-phase technique on a benzhydrylamine HCl resin in accordance with the procedures set forth in the Schedule of Preparation I.

Thus, the resin (0.5 g containing about 0.5 mmole NH$_2$) was treated during the ten successive coupling cycles with Boc-D-Ala, Boc-Pro, Boc-Leu, Boc-Arg(Tos), Boc-Lys[Z(2-Cl)], Boc-Arg(Tos), Boc-Ser(Bzl), Boc-D-Pal(3), Boc-D-Phe(4Cl), Boc-D-Nal(2) and finally with Ac$_2$O/imidazole to yield a peptide-resin which was then treated with HF and anisole to afford the free, D-Lys-containing decapeptide of III (540 mg). mg).

11

Peptides were purified on Column A with a gradient of solvent system $\underline{i}$ (20-60%B in 80 min). HPLC retention time of III was 11.4 min, when using solvent system $\underline{i}$ in a linear gradient mode (30-50% B in 20 min).

**PREPARATION IV**

Boc-D-Lys(Z$_2$-A$_2$pr)

To a mixed anhydride prepared from Z$_2$-A$_2$pr (0.72 g) and ethyl chloroformate (0.2 ml) in the presence of TEA (0.28 ml) in DMF solution (4 ml), Boc-D-Lys (0.5 g) and TEA (0.3 ml) in 50% aqueous DMF (4 ml) were added with stirring at 0°C. After 2 hours stirring at 0°C, the reaction mixture was concentrated to an oil under reduced pressure, dissolved in water and ethyl acetate, acidified with 1 M KHSO$_4$. The organic phase was washed with water, then dried over Na$_2$SO$_4$ and evaporated under vacuum to yield Boc-D-Lys-(Z$_2$-A$_2$pr) (1.1 g).

**PREPARATION V**

Ac-D-Nal(2)-D-Phe(4Cl)-D-Pal(3)-Ser-Arg-D-Lys(A$_2$pr)-Leu-Arg-Pro-D-Ala-NH$_2$          (VA)

Ac-D-Nal(2)-D-Phe(4Cl)-D-Trp-Ser-Arg-D-Lys(A$_2$pr)-Leu-Arg-Pro-D-Ala-NH$_2$          (VB)

Compounds VA and VB were built step by step on a benzhydrylamine HCl resin containing about 1 meq NH$_2$/g (Advanced ChemTech, Louisville, KY) in a reaction vessel for manual solid-phase synthesis starting with Boc-D-Ala in accordance with the procedures set forth below.

The benzhydrylamine HCl resin (1 g, about 1 mmol), after neutralization with 10% TEA in CH$_2$Cl$_2$, was coupled sequentially with 3 molar excess of protected amino acids in accordance with the Schedule given in Preparation I.

Thus, the resin was treated with Boc-D-Ala, Boc-Pro, Boc-Arg(Tos), Boc-Leu, Boc-D-Lys(Z$_2$-A$_2$pr) (Preparation IV), Boc-Arg(Tos)), Boc-Ser(Bzl), Boc-D-Pal(3), Boc-D-Phe(4Cl), and Boc-D-Nal(2). After the amino acid sequence of the decapeptide had been completed, the terminal Boc group was removed and the N-terminal was acetylated by using 10-fold excess of Ac$_2$O and imidazole to yield the peptide-resin with the structure of Ac-D-Nal(2)-D-Phe(4Cl)-D-Pal(3)-Ser(Bzl)-Arg(Tos)-D-Lys(Z$_2$-A$_2$pr)-Leu-Arg(Tos)-Pro-D-Ala-NH-RESIN. Proceeding in a similar manner but incorporating Boc D-Trp in place of Boc-D-Pal(3), the peptide-resin with the structure of Ac-D-Nal(2)-D Phe(4Cl)-D-Trp-Ser(Bzl)-Arg(Tos)-D-Lys(Z$_2$A$_2$pr)-Leu-Arg-(Tos)-Pro-D-Ala-RESIN was prepared.

The peptide-resin thus obtained was treated with anisole and HF, and the crude free peptides were isolated as described in Preparation II. Thereafter the crude peptides (1.3 g) are subjected to purification by HPLC on Column A using solvent system $\underline{i}$ in a linear gradient mode (20-50%B in 60 min).

Peptides VA and VB thus obtained (705 mg and 780 mg) were judged to be substantially (>95%) pure by using solvent system $\underline{i}$ in a linear gradient mode (30-50% B in 20 min). Retention times are 10.1 min and 17.5 min, respectively.

Alternatively, Preparation VA and VB were obtained from Preparation III and II by acylation with Boc$_2$-A$_2$pr. After purification, the Boc-protected peptides were treated with 50% TFA in DCM and repurified by HPLC (see above).

**Preparation VI**

Anthraquinone-2-methyl-hemiglutarate

576 mg (2 mmol) of 2-(hydroxymethyl)-anthraquinone was suspended in 6 ml of anhydrous pyridine and was refluxed for 24 hours with 456 mg (4 mmol) glutaric anhydride. Pyridine was eliminated under vacuum, the residue is acidified and extracted with ethyl acetate. The yellow product was recrystallized from ethyl acetate-hexane (580 mg, m.p.: 150-151 °C). HPLC retention time of Preparation VI is 19.7 min using solvent system $\underline{i}$ (linear gradient of 30-60%B in 30 min).

## EXAMPLE I

pGlu-His-Trp-Ser-Tyr-D-Lys(HMAQG)-Leu-Arg-Pro-Gly-NH$_2$    (1)

The synthesis of pGlu-His-Trp-Ser-Tyr-D-Lys(HMAQG)-Leu-Arg-Pro-Gly-NH$_2$ (3) was accomplished by coupling of [D-Lys][6]LHRH (Preparation I, 31.9 mg of the TFA salt) and anthraquinone-2-methyl-hemiglutarate (VI) with carbodiimide. The solution (200 $\mu$l DMF) of 10.6 mg anthraquinone-2-methyl-hemiglutarate and 4.6 mg HOBt was cooled down to 0°C then reacted with 3.5 $\mu$l of DIC. After 15 min, this solution was mixed with the cold solution (200 $\mu$l) of 31.9 mg [D-Lys][6]LHRH (Preparation I) (neutralized with TEA) and was kept at 0°C for 24 hours. When the reaction was not complete, the coupling was repeated with half amount of DIC. The reaction mixture was diluted with water and was subjected to HPLC on Column C using solvent system ii. Lyophilized fractions yielded 21.6 mg of peptide 3.

| HPLC data | | | |
|---|---|---|---|
| Peptide No. | Gradient (%B/min) for | | Retention time (Min) |
| | Purification | Analysis | |
| 01 | 30-50/40 | 45-65/20 | 8.6 |

## EXAMPLE II

pGlu-His-Trp-Ser-Tyr-D-Lys(MTX)-Leu-Arg-Pro-Gly-NH$_2$    (2)

Preparation of pGlu-His-Trp-Ser-Tyr-D-Lys(MTX)-Leu-Arg-Pro-Gly-NH$_2$ (2) was performed by acylating of [D-Lys][6]LHRH with methotrexate (amethopterin). To the solution of 12.0 mg of methotrexate in 100 $\mu$l of DMF equivalent of DIC was added at 0°C. After 15 min it was mixed with the neutralized (TEA) solution of 31.9 mg [D-Lys][6]LHRH (Preparation I) and was kept 0°C overnight. Thereafter the reaction mixture was diluted with water and subjected to HPLC by injection onto Column C Two main products with slightly different retention times were isolated (a: 5.2 mg, b: 5.5 mg).

| HPLC data | | | |
|---|---|---|---|
| Peptide No. | Gradient (%B/min) for | | Retention time (Min) |
| | Purification | Analysis | |
| 02 | 20-40/40 | 20-40/20 | 12.3/12.8 |

## EXAMPLE III

Other Peptides

[Ac-Nal(2)-D-Phe(4Cl)-D-Trp-Ser-Arg-D-Lys[A$_2$pr(HMAQG)$_2$]-Leu-Arg-Pro-D-Ala-NH$_2$ (3) (10.7 mg), [Ac-Nal(2)-D-Phe(4Cl)-D-Pal(3)-Ser-Arg-D-Lys[A$_2$pr(HMAQG)$_2$]-]Leu-Arg-Pro-D-Ala-NH$_2$ (4) (9.1 mg) were synthesized as described in Example I, except that [D-Lys(A$_2$pr)][6]LHRH (Preparation I, 35.9 mg), [Ac-Nal(2)[1],D-Phe(4Cl)[2],D-Trp[3],Arg[5],D-Lys(A$_2$pr)[6],D-Ala[10]]LHRH (Preparation VA, 39.6 mg) and [Ac-Nal(2)[1],D-Phe(4Cl)[2],D-Pal(3)[3],Arg[5],D-Lys(A$_2$pr)[6],D-Ala[10]]LHRH (Preparation VB, 38.8 mg) were used in a carbodiimide coupling reaction and that two times more anthraquinone-2-methyl-hemiglutarate,DIC and HOBt was used.

| HPLC data | | | |
|---|---|---|---|
| Peptide No. | Gradient (%B/min) for | | Retention time (Min) |
| | Purification | Analysis | |
| 3 | 40-80/80 | 65-85/20 | 13.9 |
| 4 | 40-70/40 | 60-80/20 | 15.2 |

**EXAMPLES IV**

Biological effects, receptor binding potencies and cytotoxic activities.

The biological effects, the receptor binding potencies and the cytotoxic activities of the claimed compounds are summarized in Table 1 to Table 4.

Table 1 shows the hormonal activity of the compounds of this invention having LHRH agonistic properties as compared to that of LHRH in dispersed rat pituitary cell superfusion system in vitro [S. Vigh and A. V. Schally, Peptides 5, 241-247 (1984)]. The peptide was infused for 3 minutes at various concentration, and the amount of LH released was compared to that released by 3 nM LHRH. Table 1 also contains data on the receptor binding affinity of these compounds for human breast cancer cell membranes.

Table 2 presents the antiovulatory activity and human breast cancer cell membrane receptor binding affinity of the claimed compounds having LHRH-inhibiting properties. The inhibitory action was determined in vivo, in 4-day cycling rats as described [A. Corbin and C. W. Beattie, Endocr. Res. Commun., 2, 1-23 (1975)].

Table 3 and 4 shows data on the inhibition of $^3$H-thymidine incorporation into DNA was by cytotoxic LHRH analogs on MCF-7, T47D, MDA-MB-231 and SKBr-3 human mammary cancer cell lines. 200,000 cells in 200 $\mu$l of RPMI-160 + 2% CFBS were incubated with 1,5 or 10 $\mu$g cytotoxic analogs for 3 hours or 23 hours then 1 $\mu$Ci $^3$H-thymidine added and incubated an additional 60 min. DNA extracted with 1 N perchloric acid and the radioactivity measured.

TABLE 1

| LH-releasing activity and receptor binding affinity of pGlu-His-Trp-Ser-Trp-R$^6$ (YX)-Leu-Arg-Pro-Gly-NH$_2$ peptides containing cytotoxic radicals for human breast cancer cell membranes. | | | | | | |
|---|---|---|---|---|---|---|
| Ex. | Peptide | | | Relative Activity | Affinity Constant** | |
| | R$^6$ | Y | X | | K$_{a1}$ nM$^{-1}$ | K$_{a2}$ $\mu$M$^{-1}$ |
| 1. | D-Lys | - | HMAQG | 35 | 1.52 | - |
| 2A. | D-Lys | - | MTX | | 5.42 | 1.59 |
| 2B. | D-Lys | - | MTX | | 0.63 | - |

─* LH-releasing activity was compared to that produced by 3 nM LH-RH.
**$^{125}$I-[D-Trp]$^6$ LHRH used as labelled ligand.

TABLE 2

| Antiovulatory activity and affinity of Ac-D-Nal (2)-D-Phe(4Cl)-$R^3$-Ser-$R^5$-D-Lys[AX]-Leu-Arg-Pro-D-Ala-$NH_2$ peptides containing cytotoxic radicals for membrane receptors of human breast cancer cells. | | | | | | |
|---|---|---|---|---|---|---|
| Ex. | Peptide | | | | %Ovulation Blockade* | Affinity Constant** | |
| | $R^3$ | $R^5$ | A | X | | $K_{a1}$ $nM^{-1}$ | $K_{a2}$ $uM^{-1}$ |
| 3. | D-Trp | Arg | L-$A_2$pr | (HMAQG)$_2$ | 0 | 7.05 | 8.6 |
| 4. | D-Pal(3) | Arg | L-$A_2$pr | (HMAQG)$_2$ | 40 | 2.28 | - |

* Peptides were tested at 10 $\mu$g per rat.

**$^{125}$I-[D-Trp][6] LHRH used as the labelled ligand

NB, no binding

TABLE 3

| Inhibitory effect of cytotoxic LHRH analogs of Formula I on $^3$H-Thymidine incorporation into DNA in MCF-7 human breast cancer cell line. | | | |
|---|---|---|---|
| Ex. | Dose $\mu$g/ml | % Inhibition at 4 hrs | % Inhibition at 24 hrs |
| Control | | 0 | 0 |
| 1 | 1<br>10 | 29**<br>32** | 14**<br>71** |
| 3 | 1<br>10 | | 3<br>11 |
| 4 | 1<br>10 | 31**<br>28** | 15<br>40** |

**p<0.01 by Duncan's multiple range test.

## TABLE 4

### Inhibitory effect of cytotoxic LHRH analogs of Formula I on $^3$H-Thymidine incorporation into DNA in different human breast cancer cell lines.

| Ex. | Dose $\mu$g/ml | % Inhibition at 4 hrs | % Inhibition at 24 hrs |
|---|---|---|---|
| **T47D Cell line** | | | |
| Control | - | 0 | 0 |
| 2 | 1 | 38** | 26 |
| | 10 | 54** | 41 |
| 3 | 1 | 37** | 20 |
| | 10 | 41** | 54 |
| **MDA-MB-231 Cell Line** | | | |
| Control | - | 0 | 0 |
| 2 | 1 | 23* | 0 |
| | 10 | 31** | 8 |
| 3 | 1 | 36** | 0 |
| | 10 | 40** | 90** |
| **SKBr-3 Cell line** | | | |
| Control | - | 0 | 0 |
| 2 | 1 | 21** | 16** |
| | 10 | 36** | 10 |
| 3 | 1 | 30** | 9 |
| | 10 | 53** | 88** |

*p<0.05 by Duncan's multiple range test.

**p<0.01 by Duncan's multiple range test.

**ABSTRACT**

The present invention deals with LHRH analogs which contain cytotoxic moieties, have influence on the release of gonadotropins from the pituitary in mammals (possess high agonistic or antagonistic activity) and have antineoplastic effect. The compounds of this invention are represented by Formula I: X-R$^1$-R$^2$-R$^3$-Ser-R$^5$-R$^6$(Q)-Leu-Arg-Pro-R$^{10}$-NH$_2$, wherein R$^1$ is pGlu or D-Nal(2), R$^2$ is His or, provided that R$^1$ is D-Nal(2), R$^2$

is D-Phe(4Cl), $R^3$ is Trp or, provided that $R^1$ is D-Nal(2), $R^3$ is D-Trp or D-Pal(3), $R^5$ is Tyr, or, provided that $R^1$ is D-Nal(2), $R^5$ is Arg, $R^6$ is D-Lys, $R^{10}$ is Gly or, provided that $R^1$ is D-Nal(2), $R^{10}$ is D-Ala, X is hydrogen or, provided that $R^1$ is D-Nal(2), X is acetyl, Q is a cytotoxic moiety having the formula -$Q^4$ or -A-($Q^2$) or provided that $R^1$ is D-Nal(2), Q is -B(A$Q^2$)$_2$, wherein A is glutaryl, B is 2,3-diaminopropionyl, the -CO moiety of A- and of B- being bonded to an amino group on $R^6$, and in the group B(A$Q^2$)$_2$, the -CO moiety of A-being bonded to an amino group on B, $Q^2$ is anthraquinonylmethoxy, $Q^4$ is methotrexoyl and pharmaceutically acceptable salts thereof and methods of use pertaining these compounds.

**Claims**

**Claims for the following Contracting States : AT, BE, CH, DE, DK, FR, GB, IT, LI, LU, NL, SE**

**1.** A peptide of the formula

pGlu-His-Trp-Ser-Tyr-D-Lys(Q)-Leu-Arg-Pro-Gly-NH$_2$

wherein Q is a cytotoxic moiety consisting of methotrexoyl or anthraquinone-2-methylglutaryl.

**2.** A peptide of the formula

Ac-D-Nal(2)-D-Phe(4 Cl)-R$^3$-Ser-Arg-D-Lys [A$_2$pr (HMAQG)$_2$] - Leu-Arg-Pro-D-Ala-NH$_2$

wherein $R^3$ is D-Trp or D-Pal(3).

**3.** Use of any of the peptides of claims 1 and 2 or a pharmaceutically acceptable salt thereof for the preparation of a composition for the treatment of cancers.

**Claims for the following Contracting States : ES, GR**

**1.** A process for the production of a peptide of the formula

pGlu-His-Trp-Ser-Tyr-D-Lys(Q)-Leu-Arg-Pro-Gly-NH$_2$

wherein Q is a cytotoxic moiety consisting of methotrexoyl or anthraquinone-2-methylglutaryl by reacting an intermediate peptide of the formula pGlu-His-Trp-Ser-Tyr-D-Lys-Leu-Arg-Pro-Gly-NH$_2$
a) in case of methotrexoyl as cytotoxic moiety with methotrexate, and
b) in case of anthraquinone-2-methylglutaryl as cytotoxic moiety in a first version with anthraquinone-2-methyl-hemiglutarate or in a second version with an acylating agent with a glutaryl moiety and then coupling the obtained product with 2-(hydroxymethyl)-anthraquinone.

**2.** A process for the production of a peptide of the formula

Ac-D-Nal(2)-D-Phe(4 Cl)-R$^3$-Ser-Arg-D-Lys [A$_2$pr (HMAQG)$_2$] - Leu-Arg-Pro-D-Ala-NH$_2$

wherein $R^3$ is D-Trp or D-Pal(3) by coupling an intermediate peptide of the formula Ac-D-Nal(2)-D-Phe(4 Cl)-R$^3$-Ser-Arg-D-Lys(A$_2$pr)-Leu-Arg-Pro-D-Ala-NH$_2$ in a first version with anthraquinone-2-methyl-hemiglutarate or reacting said intermediate peptide in a second version with an acylating agent with a glutaryl moiety and then coupling the obtained product with 2-(hydroxymethyl)-anthraquinone.

**Patentansprüche**

**Patentansprüche für folgende Vertragsstaaten : AT, BE, CH, DE, DK, FR, GB, IT, LI, LU, NL, SE**

**1.** Peptid der Formel

pGlu-His-Trp-Ser-Tyr-D-Lys(Q)-Leu-Arg-Pro-Gly-NH$_2$

worin Q ein cytotoxischer Rest, bestehend aus Methotrexoyl oder Antrachinon-2-methylglutaryl, ist.

**2.** Peptid der Formel

Ac-D-Nal(2)-D-Phe(4Cl)-R$^3$-Ser-Arg-D-Lys[A$_2$pr(MHAQG)$_2$]-Leu-Arg-Pro-D-Ala-NH$_2$

worin R$^3$ D-Trp oder D-Pal(3) ist.

**3.** Verwendung der Peptide gemäß Ansprüchen 1 und 2 oder deren pharmazeutisch brauchbaren Salze zur Herstellung einer Zusammensetzung für die Krebsbehandlung.

**Patentansprüche für folgende Vertragsstaaten : ES, GR**

**1.** Verfahren zur Herstellung eines Peptids mit der Formel

pGlu-His-Trp-Ser-Tyr-D-Lys(Q)-Leu-Arg-Pro-Gly-NH$_2$,

in welcher Q ein cytotoxischer Methotrexoyl- oder Anthrachinon-2-methylglutaryl-Rest ist, in dem man ein Peptid-Zwischenprodukt der Formel pGlu-His-Trp-Ser-Tyr-D-Lys-Leu-Arg-Pro-Gly-NH$_2$
a) im Falle eines cytotoxischen Methotrexoyl-Rests mit Methotrexat reagieren läßt, und
b) im Falle eines cytotoxischen Anthrachinon-2-methylglutaryl-Rests nach einer ersten Variante mit Anthrachinon-2-methylhemiglutarat oder nach einer zweiten Variante mit einem einen Glutaryl-Rest enthaltenden Acylierungsmittel und sodann das erhaltene Produkt mit 2-Hydroxymethyl-anthrachinon koppelt.

**2.** Verfahren zur Herstellung eines Peptids der Formel

Ac-D-Nal(2)-D-Phe(4 Cl)-R$^3$-Ser-Arg-D-Lys [A$_2$pr (HMAQG)$_2$] - Leu-Arg-Pro-D-Ala-NH$_2$,

in welcher R$^3$ D-Trp oder D-Pal(3) ist, in dem man ein Peptid-Zwischenprodukt der Formel Ac-D-Nal(2)-D-Phe(4 Cl)-R$^3$-Ser-Arg-D-Lys(A$_2$pr)-Leu-Arg-Pro-D-Ala-NH$_2$ nach einer ersten Variante mit Anthrachinon-2-methylhemiglutarat reagieren läßt oder dieses Peptid-Zwischenprodukt nach einer zweiten Variante mit einem einen Glutaryl-Rest enthaltenden Acylierungsmittel reagieren läßt und sodann das erhaltene Produkt mit 2-Hydroxymethyl-anthrachinon koppelt.

## Revendications
**Revendications pour les Etats contractants suivants : AT, BE, CH, DE, DK, FR, GB, IT, LI, LU, NL, SE**

**1.** Peptide de formule :

pGlu-His-Trp-Ser-Tyr-D-Lys(Q)-Leu-Arg-Pro-Gly-NH$_2$

dans lequel Q est une partie cytotoxique consistant en méthotrexoyle ou anthraquinone-2-méthylglutaryle.

**2.** Peptide de formule :

Ac-D-Nal(2)-D-Phe(4Cl)-R$^3$-Ser-Arg-D-Lys[A$_2$pr(HMAQG)$_2$]-Leu-Arg-Pro-D-Ala-NH$_2$

dans lequel R$^3$ est D-Trp ou D-Pal(3).

**3.** Utilisation de l'un quelconque des peptides des revendications 1 et 2, ou d'un sel pharmaceutiquement acceptable de ceux-ci, dans la préparation d'une composition pour le traitement de cancers.

**Revendications pour les Etats contractants suivants : ES, GR**

**1.** Procédé de production d'un peptide répondant à la formule

pGlu-His-Trp-Ser-Tyr-D-Lys(Q)-Leu-Arg-Pro-Gly-NH$_2$

dans laquelle Q représente un groupe cytotoxique méthotrexoylique ou anthraquinone-2-méthylglutaryque, en faisant réagir un peptide intermédiaire obéissant à la formule pGlu-His-Trp-Ser-Tyr-D-Lys-Leu-Arg-Pro-Gly-NH$_2$

a) avec le méthotrexate au cas où l'on utilise un groupe cytotoxique méthotrexoylique, et

b) au cas où l'on utilise un groupe cytotoxique anthraquinone-2-méthylglutarylique, d'après une première réalisation, avec l'anthraquinone-2-méthyl-hémiglutarate, ou d'après une seconde réalisation, avec un agent d'acylation comportant un groupe glutarylique et puis en combinant le produit obtenu à la 2-(hydroxyméthyl)-anthraquinone.

2. Procédé de production d'un peptide répondant à la formule

Ac-D-Nal(2)-D-Phe(4 Cl)-R$^3$-Ser-Arg-D-Lys [A$_2$pr (HMAQG)$_2$] - Leu-Arg-Pro-D-Ala-NH$_2$

dans laquelle R$^3$ représente D-Trp ou D-Pal(3) en combinant un peptide intermédiaire répondant à la formule Ac-D-Nal(2)-D-Phe(4 Cl)-R$^3$-Ser-Arg-D-Lys(A$_2$pr)-Leu-Arg-Pro-D-Ala-NH$_2$

d'après une première réalisation à l'anthraquinone-2-méthyl-hémiglutarateou, d'après une seconde réalisation, en faisant réagir ledit peptide intermédiaire avec un agent d'acylation comportant un groupe glutarylique et puis combinant le produit obtenu à la 2-(hydroxyméthyl)-anthraquinone.